# EUROPÄISCHE PATENTANMELDUNG

(11) **EP 2 210 624 A2**
(43) Veröffentlichungstag der Anmeldung: **28.07.2010**
(21) Anmeldenummer: 10150449.6
(22) Anmeldetag: 11.01.2010
(51) Int. Cl.: A61L 27/42, A61L 27/56

(54) **Isoelastische, bioverträgliche Implantatwerkstoffe**

(30) Priorität: 17.01.2009 DE 102009005031
(71) Anmelder: Deutsches Zentrum für Luft- und Raumfahrt e.V., 51147 Köln (DE)
(72) Erfinder: Prof. Ratke, Lorenz, 53757, St. Augustin (DE)
(74) Vertreter: von Kreisler Selting Werner

(57) **Zusammenfassung**

Die Erfindung betrifft ein endoprothetisches Implantat aus einer Metallmatrix mit eingebetteten nanostrukturierten Materialien, ein Verfahren zu dessen Herstellung sowie Verwendung desselben.

## Beschreibung

Die Erfindung betrifft ein endoprothetisches Implantat aus einer Metallmatrix mit eingebetteten nanostrukturierten Materialien, ein Verfahren zu dessen Herstellung sowie Verwendung desselben.

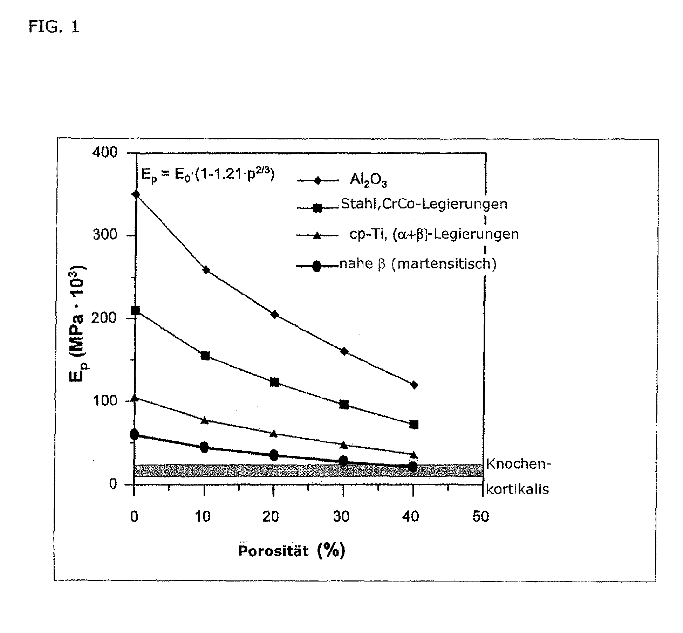

Für Implantate aus dem Bereich der Endoprothetik werden heute üblicherweise Ti-Basislegierungen verwendet, da sie die beste Kompatibilität zum menschlichen Körper haben (keine Korrosion, keine Abgabe von metallischen Spurenelementen, die im menschlichen Körper angereichert werden). Nachteil aller metallischen Legierungen ist unabhängig von der Bioverträglichkeit, dass der E-Modul um mehr als einen Faktor zehn höher ist als der des menschlichen Knochens. Dies kann nach der Implantation zum Effekt des mechanischen Abschirmens des Knochens durch das Implantat und somit zur Störung des Gleichgewichts im Knochengewebe zwischen spannungsinduzierten Auf-und Abbau führen. Man versucht der elastischen Inkompatibilität durch geschicktes CAD-Design der Prothesen, beispielsweise für Hüfte, Knie oder Schulter entgegenzuwirken. Dem sind ebenso prinzipielle Grenzen gesetzt, wie der Variation des E-Moduls durch legierungstechnische Maßnahmen. Der E-Modul von reinem cp-Ti (unlegiertes, kommerziell reines Titan) mit 105 GPa ist sehr ähnlich dem von Ti6Al4V, Ti6Al7Nb. Einzig extreme Zulegierung wie bei Ti12Mo6Zr2Fe und Ti30Ta erzeugen E-Module im Bereich von 65 bis 80 GPa. Diese Werte sind immer noch ca. 5-6 Mal höher als der der Knochenkortikalis (10 bis 20 GPa). Ein bekannter Ansatz geht drauf zurück, dass poröse Körper einen niedrigeren E-Modul haben als massive Körper. In Jef A. Helsen, H. Jürgen Breme (Eds.), Metals as biomaterials, Wiley 1998 (Kap. 1 und 5) wird mit Hilfe der Beziehung Eₚ=E₀(1-1.21p^{2/3}), wobei Eₚ den E-Modul des porösen Körpers bezeichnet, E₀ des massiven Materials und p die Porosität ist, für verschiedene Implantatwerkstoffe berechnet, wie theoretisch der E-Modul durch eine ausreichend hohe Porosität auf den Wert des Knochens eingestellt werden kann. Fig. 1 zeigt das Ergebnis der Rechnung, das heißt den E-Modul als Funktion der Porosität für verschiedene Werkstoffe.

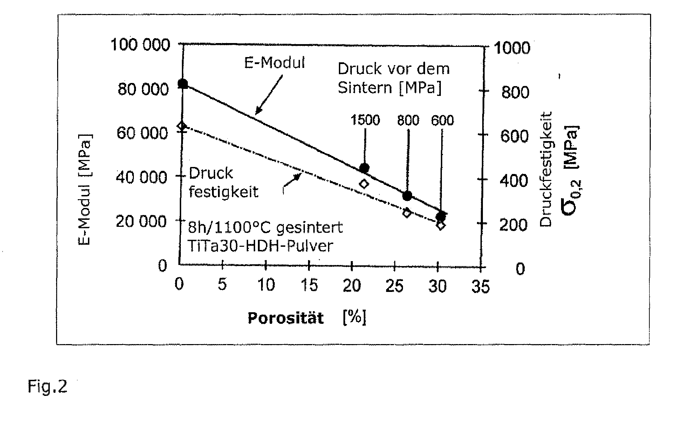

Experimentell wurde diese Idee von Jef A. Helsen loc. cit. aufgegriffen und für Zahnimplantate entwickelt. Fig. 2 zeigt, dass die Idee grundsätzlich richtig ist und ein Werkstoff erzielt werden kann, dessen E-Modul nur noch 100% entfernt ist vom E-Modul eines menschlichen Knochens. In Fig. 2 wird der gemessene E-Modul und die Druckfestigkeit gegen die Porosität aufgetragen am Beispiel der Legierung Ti30Ta. Die so hergestellten Implantate haben aber einen entscheidenden Nachteil: sie sind offenporige Werkstoffe. Es ist bekannt, dass deren Porendurchmesser groß genug sein muss, damit eine genügende Vaskularisierung des einwachsenden Gewebes gewährleistet ist. Ist die Blutversorgung ungenügend, kann es zu einem Versagen des Implantats kommen (beispielsweise Osteolyse). Dies wurde häufig bei Implantaten mit entsprechend rauer Oberfläche beobachtet.

DE 10 2006 009 917 A1 beschreibt einen porösen Verbundwerkstoff aus einer Metallmatrix mit eingebetteten nanostrukturierten Materialien. Das Problem der Herstellung "isoelastischer", insbesondere bioverträglicher Implantatwerkstoffe ist jedoch trotz guter Ansätze nicht gelöst.

Die Nachteile des Standes der Technik bestehen somit in der Osteolyseproblematik offenporiger Werkstoffe, der geringen Porosität von kleiner 50%, als theoretisch notwendig wäre und die starke Differenz der E-Moduli von Knochen zu Implantat.

Die Aufgabe wird erfindungsgemäß dadurch gelöst, das endoprothetische Implantate aus einer Metallmatrix mit eingebetteten nanostrukturierten Materialien eingesetzt werden.

Erfindungsgemäß werden syntaktische Implantate aus einem geeigneten Aerogelgranulat und einer handelsüblichen Implantat-Titanlegierung eingesetzt. Da Titanschmelzen extrem reaktiv sind und keine Reaktion mit dem Aerogel bei den hohen Abgusstemperaturen erfolgen dürfen, werden Aerogele und Xerogele aus Titanoxid verwendet, die vollständig kompatibel mit Titanlegierungen sind. Ebenso können hochschmelzende Aerogele oder Xerogele aus Zirkonoxid verwendet werden.

Poren im Sinne der Erfindung sind solche Volumenbereiche des Implantats, die nicht von Metall ausgefüllt sind und eine Dichte in einem Bereich von 0,001 g/cm³ bis 0,1 g/cm³ aufweisen. Die Poren können vorteilhafterweise teilweise oder vollständig durch die eingebetteten nanostrukturierten Materialien gefüllt sein. Die erfindungsgemäße Bezeichnung Poren, die klassischerweise mit Gas gefüllt sind, weicht also bewusst vom bisherigen Verständnis ab, da die erfindungsgemäßen Poren auch beispielsweise mit Feststoffen wie Aerogel ausgefüllt sein können.

Nanostrukturierte Materialien, insbesondere Aerogelgranulate im Sinne der Erfindung umfassen solche, die Erhebungen auf ihrer Oberfläche aufweisen, von denen mindestens 80 % der Erhebungen einen Abstand von benachbarten Erhebungen im Bereich von 5 nm bis 500 nm aufweisen, wobei die Erhebungen selbst eine Höhe in einem Bereich von 5 nm bis 500 nm besitzen. Zudem sind darunter Materialien zu verstehen, deren innere Struktur aus Nanoteilchen besteht, also Teilchen mit einem Durchmesser in einem Bereich von 2 bis 100 nm, die vernetzt sind. Liegen die nanostrukturierten Materialien als Teilchen vor, so liegt die Teilchengröße vorteilhafterweise in einem Bereich von 0,1 bis 5 mm.

Vorteilhafterweise liegt die Porosität des erfindungsgemäßen Implantats in einem Bereich von 20 bis 80 %, insbesondere bevorzugt in einem Bereich von 30 bis 70 %. Die Porosität im Sinne der Erfindung ist das Verhältnis des Gewichts eines bestimmten vorgegebenen Volumens des erfindungsgemäßen Implantats zu dem Gewicht eines entsprechend porenfreien Metallkörpers desselben Volumens. Ist die Porosität zu groß, so weist das Implantat eine zu geringe mechanische Festigkeit auf. Ist die Porosität zu gering, so ist das Gewicht und der E-Modul dieses Implantats zu hoch. Dadurch dass die Poren vorteilhafterweise auch durch die nanostrukturierten Materialien gefüllt sein können, entspricht in diesem Fall die Porosität also im Wesentlichen dem Volumengehalt der nanostrukturierten Materialien, für den Fall, dass die nanostrukturierten Materialien vernachlässigbar leicht sind.

Das Volumen der einzelnen gefüllten Poren ist bevorzugt so eingestellt, dass das Volumen von mindestens 80 % der Poren höchstens jeweils 125 mm³ beträgt. Beträgt das Volumen von mehr als 80 % der Poren jeweils mehr als 125 mm³, so sind diese Implantate nicht hinreichend mechanisch belastbar. Die Porengröße des erfindungsgemäßen Verbundwerkstoffs kann beispielsweise nach ASTM 3576-77 bestimmt werden.

Vorteilhafterweise sind die nanostrukturierten Materialien chemisch inert. Chemisch inert im Sinne der Erfindung bedeutet, dass die nanostrukturierten Materialien keine chemische Reaktion mit geschmolzenem Metall eingehen. Dies ist besonders vorteilhaft, da so eine Degradierung, beispielsweise Oxidation, der Metallmatrix vermieden werden kann.

Die nanostrukturierten Materialien sind vorzugsweise Aerogele auf der Basis von Titandioxid und/oder Zirkondioxid. Durch die geringe Dichte dieser Materialien können bei der Herstellung Partikel oder auch Granulate dieser Materialien mit metallischen Schmelzen umgossen werden und bilden so die Poren des erfindungsgemäßen Implantats, ohne Notwendigkeit, diese Materialien aus dem Implantat zu entfernen. Dies gilt insbesondere für Aerogelgranulate, da die Dichte des erfindungsgemäß eingesetzten Aerogelgranulats vorteilhafterweise in einem Bereich von 0,005 bis 0,025 g/cm³ liegt. Aerogel ist besonders vorteilhaft, da es offenzellig ist und eine hohe spezifische Oberfläche besitzt.

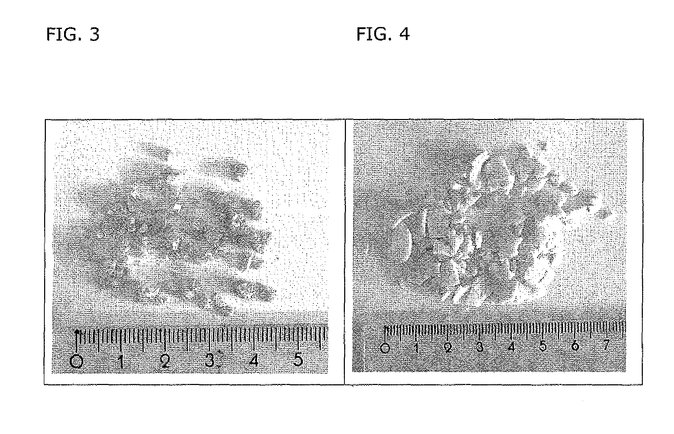

Der Porendurchmesser des Aerogels selbst liegt vorteilhafterweise in einem Bereich von 5 bis 50 nm. Die spezifische Oberfläche der eingesetzten erfindungsgemäßen Aerogele liegt vorteilhafterweise in einem Bereich von 200 bis 1500 m²/g. Vorteilhafterweise liegt die Wärmeleitfähigkeit der Aerogele in einem Bereich von 0,005 bis 0,03 W/mK bei 25 °C. Das Aerogel liegt vorzugsweise als Granulat vor, insbesondere als Granulat, bei dem die Korngrößenverteilung dergestalt ist, dass mindestens 80 Vol.-% des Aerogelgranulats eine Körnung in einem Bereich von 0,1 bis 5 mm aufweist, wie dies in den Fig. 3 und 4 dargestellt ist. Beide Fig. zeigen dabei ein Titanoxidaerogelgranulat. Die Form der Körner des Aerogels ist vorteilhafterweise ausgewählt aus kugelförmig, polyedrisch, zylindrisch oder plättchenförmig.

Das Metall der Matrix ist erfindungsgemäß ausgewählt aus Titan oder einer an sich im Stand der Technik bekannten Legierung aus mindestens einem weiteren Metall.

Die erfindungsgemäßen Implantate weisen vorteilhafterweise eine Stauchhärte beziehungsweise Druckfestigkeit bei einer Stauchung von 20 % von mindestens 8 MPa auf (nach DIN 53577 / ISO 3386). Das Raumgewicht der erfindungsgemäßen Implantate liegt vorteilhafterweise in einem Bereich von 0,3 bis 2 g/cm³, insbesondere in einem Bereich von 1 bis 2 g/cm³.

In einer weiteren Ausführungsform wird die der Erfindung zugrundeliegende Aufgabe gelöst durch ein Verfahren zur Herstellung des erfindungsgemäßen Implantats, das dadurch gekennzeichnet ist, dass man folgende Schritte durchführt:
a) externes Mischen des nanostrukturierten Materials mit einer Metallschmelze und Überführen in eine Gussform oder
a') Mischen des nanostrukturierten Materials mit einer Metallschmelze in der Gussform,
b) Erstarren lassen, und
c) Entnahme aus der Form.

Alternativ ist es auch möglich, die nanostrukturierten Materialien mit einem Metallpulver zu mischen und anschließend das Metall zu schmelzen.

Die Aufgabe wird also dadurch gelöst, dass insbesondere Aerogelteilchen in eine gegebenenfalls thixotrope Metallschmelze eingerührt werden. Da das Aerogel chemisch inert ist, findet keine Reaktion zwischen dem Metall und der Schmelze statt. Während des Rührens erstarrt das Metall und schließt die Aerogelteilchen ein. Noch im weichen Zustand kann der Metallverbund vorteilhafterweise gepresst werden, so dass eine gewünschte Formgebung erfolgen kann. Thixotrop im Sinne der Erfindung ist die Metallschmelze immer dann, wenn deren Temperatur zwischen der Liquidus- und Solidustemperatur liegt.

Das Verfahren kann auch vorteilhafterweise auf dem Hinterfüllen einer Anhäufung von Aerogelgranulat mit einer Metallschmelze beruhen. Die vorteilhafterweise druckbeaufschlagte Schmelze dringt in die Zwischenräume ein und füllt die Zwickel aus. Nach der Erstarrung wird das Aerogel nicht entfernt, da es mit einer Dichte von beispielsweise etwa 0,015 g/cm³ nur einen Bruchteil des Gesamtgewichts ausmacht. Die Druckbeaufschlagung kann vorteilhafterweise bei kleineren Bauteilen durch die Zentrifugalkraft im Schleuderguss realisiert werden und bei größeren Bauteilen im Druckguss.

In einer weiteren Ausführungsform wird die der Erfindung zugrundeliegende Aufgabe gelöst durch die Verwendung der erfindungsgemäßen Verbundwerkstoffe als künstliches Hüftgelenk, Kniegelenk, Schultergelenk, Sprunggelenk, Ellenbogengelenk oder Fingergelenk.

Das erfindungsgemäße Titan-Implantat ist über die Porosität im elastischen Modul so einstellbar, dass eine Isoelastizität zum Knochen möglich ist.

Da keine offenen Poren existieren, sondern diese alle mit einem biokompatiblen Aerogel gefüllt sind, gibt es kein Problem der Osteolyse durch ineffiziente Vaskularisierung.

Ausführungsbeispiel:

### 1. Herstellung der Titanoxidaerogel-Granulate

Zur Herstellung von Titandioxid-Aerogelen wurde ein Sol-Gel Prozess angewandt. Dazu wurden zwei Lösungen mit folgenden Mengen, jeweils in g angesetzt:

| Lösung 1 | | Lösung 2 | | |
|---|---|---|---|---|
| deion. H₂O | 1-Butanol | TBOT | 1-Butanol | HCl 32% |
| 4,53 | 35,99 | 22,65 | 19,23 | 2,45 |

Als Alkoxid wurde TBOT=Tetra-n-butylorthotitanat (Merck) verwendet. 1-Butanol war von Fluka und HCl (32%) von Merck. Gleiche Volumenanteile von Lösung 1 und Lösung 2 wurden vermischt. Gelierzeit betrug ca. 1-2 min. Danach wurde das nasse Gel für 2-3 Tage gealtert und dann in ein 2-Propanolbad für eine Woche eingelegt. Danach wurden die nassen Gele getrocknet. Dieses konnte entweder überkritisch oder unterkritisch geschehen:

Für die überkritische Trocknung wurde 2-Propanol in eine Autoklavenwanne gefüllt, in die die Gele getaucht wurden. Propanol wurde in mehreren Waschzyklen gegen CO₂ ausgetauscht. Nach vollständigem Austausch wurde überkritisch getrocknet indem die Temperatur auf 40°C erhöht wurde und sich dabei ein Druck von ca. 90 bar einstellte. Im überkritischen Bereich ruhen die Gele mindestens 2 Stunden bevor der Druck bei konstanter Temperatur im Laufe von 4 Stunden auf 1 bar abgesenkt wurde.

Für die unterkritische Trocknung wurde der Behälter mit dem nassen Gel mit einem Parafilm^{®} (durchscheinende Verschlussfolie aus etwa gleichen Teilen Paraffin-Wachs und Polyethylen) abgedeckt, der gelöchert wurde, dass sich eine geeignete Verdampfungsrate ergab. Ein Trocknen von Proben mit ca. 50 ml Volumen in 4 bis 6 Wochen erwies sich als gut. Trotz langsamen Trocknens riss das nasse Gel in Stücke.

Die so erzeugten Aerogele und Xerogele bestanden aus Chips, die ca. 1-3 mm Dicke und unregelmäßige Berandung hatten. Sie wurden mit beispielsweise einem Mörser grob granuliert (siehe Fig. 3 und Fig. 4).

### 2. Herstellung der Zirkonoxidaerogele

Zur Herstellung von Zirkonoxidaerogelen und Xerogelen konnten zwei Methoden verwendet werden.

Bei der ersten Methode wurden 24,7 ml Tetra-n-Butoxy-Zirkonium mit 45 ml Ethanol und 3,1 ml konzentrierter Salpetersäure (s.o.) vermischt und mit einer Lösung aus 3,4 ml deionisertem Wasser und 22,5 ml Ethanol unter kontinuierlichem Rühren vermischt. Innerhalb weniger Sekunden gelierte die Lösung. Das nasse Alkogel wurde für einen Tag in Ethanol gelagert (Alterung) und konnte dann entweder unterkritisch getrocknet werden (siehe Titanoxidxerogele) oder überkritisch nach Lösungsmittelaustausch, insbesondere mit Kohlendioxid.

Bei der zweiten Methode wurden 14,3 g Zirkonchlorid mit 103 ml deionisertem Wasser gemischt und dann 39,4 ml Propylenoxid hinzugegeben. Innerhalb von 2 Minuten gelierte die Lösung und es konnte unterkritisch oder überkritisch getrocknet werden. Beide Verfahren sind an sich aus der Literatur bekannt.

Die Aerogele aus Titanoxid wie auch Zirkoniumoxid wiesen typischerweise spezifische Oberflächen von 300 bis 500 m²/g, Dichten im Bereich 0,2 bis 0,5 g/cm³ (amorphes TiO₂) und 0,3 bis 0,6 g/cm³ für amorphes Zirkonia auf. Die Titanoxidxerogele hatten typischerweise eine Dichte zwischen 30 und 40% der Dichte des jeweiligen kristallinen Materials, abhängig von der Trocknungsgeschwindigkeit.

### Herstellung des Gussteils:

Der Implantatverbundwerkstoff wurde hergestellt, in dem in eine Form das beschriebene Granulat eingefüllt wurde und eine Titanschmelze im Schleudergussverfahren die Zwischenräume des Granulats infiltriert. Auf diese Weise konnten Verbundwerkstoffe mit bis zu 70 Vol.% Aerogel/Xerogel im Werkstoff hergestellt werden.

Benutzt man nicht den Schleuderguss, sondern saugte die Schmelze aus einem Kaltschmelztiegel in die mit Aerogel/Xerogel gefüllte Gussform, sind Gussteile herstellbar mit Aerogelgehalten zwischen 40 und 60 Volumenprozent.

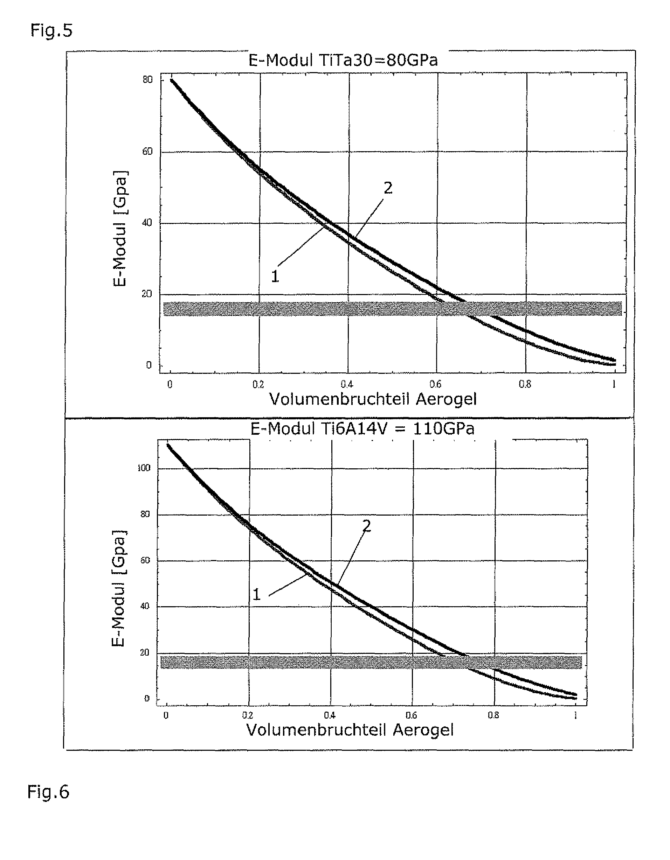

Beispiele für die möglichen E-Module bei zwei verschiedenen Dichten der Aerogele (0,2 und 0,6g/cm³) zeigen die Fig. 5 und 6, wobei der Bereich für den E-Modul von Knochen grau unterlegt wurde. Die beiden Linien in Fig. 5 zeigen den theoretischen Verlauf des E-Moduls für zwei verschiedene Aerogeldichten ([1] = 0,2 g/cm³, [2] = 0,6 g/cm³), wenn die Basislegierung TiTa30 ist. Die beiden Linien in Fig. 6 zeigen den theoretischen Verlauf des E-Moduls für zwei verschiedene Aerogeldichten ([1] = 0,2 g/cm³, [2] = 0,6 g/cm³), wenn die Basislegierung Ti6AlV4 ist.

Da Xerogele eine deutliche höhere Dichte als Aerogele haben (bei Titanoxid ca. 1,25 g/cm³) wurde ein deutlich höherer Anteil benötigt, um einen isoelastischen Zustand zu erreichen. Nach dem selben Modell berechnete sich ein Wert von ca. 80 Vol.%. Nach den bisherigen Versuchen war ein so hoher Volumenanteil schwer zu erreichen.

Die Fig. 5 und 6 zeigen, dass mit einem Aerogel-Volumengehalt im Bereich von 50 bis 70 Vol.-% Werkstoffe herstellbar waren, die isoelastisch zum Knochen sind (Berechnung nach L. Gibson, M. Ashby, Cellular Solids, Cambridge University Press, 2nd Ed., Cambridge UK, 1997, Chapter 5), abhängig vom E-Modul der Basislegierung.

## Patentansprüche

1. Endoprothetisches Implantat aus einer Metallmatrix mit eingebetteten nanostrukturierten Materialien.

2. Endoprothetisches Implantat gemäß Anspruch 1, **dadurch gekennzeichnet, dass** die Metallmatrix Titan oder eine Titanlegierung umfasst.

3. Endoprothetisches Implantat nach Anspruch 1 oder 2, **dadurch gekennzeichnet, dass** die nanostrukturierten Materialien chemisch inert sind.

4. Endoprothetisches Implantat gemäß einem der vorherigen Ansprüche 1 bis 3, **dadurch gekennzeichnet, dass** die nanostrukturierten Materialien Titanoxid und/oder Zirkonoxidaerogele umfassen.

5. Endoprothetisches Implantat gemäß Anspruch 1, **dadurch gekennzeichnet, dass** die Porosität in einem Bereich von 20 bis 80 % liegt.

6. Verfahren zur Herstellung eines endoprothetischen Implantats gemäß einem der Ansprüche 1 bis 5, **dadurch gekennzeichnet, dass** man folgende Schritte durchführt:
a) externes Mischen des nanostrukturierten Materials mit einer Metallschmelze und Überführen in eine Gussform oder a') Mischen des nanostrukturierten Materials mit einer Metallschmelze in der Gussform,
b) Erstarren lassen, und
c) Entnahme aus der Form.

7. Verwendung des endoprothetischen Implantats gemäß einem der Ansprüche 1 bis 6 als künstliches Hüftgelenk, Kniegelenk, Schultergelenk, Sprunggelenk, Ellenbogengelenk oder Fingergelenk.
